Europäisches Patentamt

European Patent Office

Office européen des brevets

(11) Publication number: **0 431 164 A1**

# EUROPEAN PATENT APPLICATION
## published in accordance with Art. 158(3) EPC

(21) Application number: 89909892.5

(22) Date of filing: 21.04.89

(86) International application number:
PCT/SU89/00106

(87) International publication number:
WO 90/12584 (01.11.90 90/25)

(51) Int. Cl.5: **A61K 35/50**

(43) Date of publication of application:
12.06.91 Bulletin 91/24

(84) Designated Contracting States:
DE FR GB IT NL

(71) Applicant: KISHINEVSKY
SELSKOKHOZYAISTVENNY INSTITUT IMENI
M.V. FRUNZE
ul. Gribova, 44
Kishinev, 277049(SU)

Applicant: RESPUBLIKANSKY
MEDIKO-PEDAGOGICHESKY TSENTR BRAKA
I SEMII
ul. Korolenko, 2
Kishinev, 277028(SU)

(72) Inventor: REILIAN, Nikon Semenovich
ul. Zelinskogo, 28/3-33
Kishinev, 277038(SU)
Inventor: GOLBAN, Dmitry Maximovich
ul. Belskogo, 42-75
Kishinev, 277060(SU)

(74) Representative: Wain, Christopher Paul et al
A.A. THORNTON & CO. Northumberland
House 303-306 High Holborn
London WC1V 7LE(GB)

(54) **PREPARATION WITH AN ANTI-INFLAMMATORY, LACTOGENOUS AND STIMULATING ACTION, AND METHOD OF OBTAINING IT.**

(57) A preparation with an anti-inflammatory, lactogenous and stimulating action consists of a water emulsion of human placental chorion denatured by chlorine having a pH of 2.5 to 3.0 and a solid-particle size not exceeding 0.4 mm, a solid residue of 1.70 to 2.3 % by weight, and containing, per 1 ml, no less than 0.05 mg of nucleic acids, no less than 6.0 mg of protein and no less than 0.30 mg of hexuronic acids. A method of obtaining said preparation consists in that the placenta chorion is washed, disintergrated then denatured by treating it with a solution of chlorine in distilled water at a concentration of the chlorine not exceeding 0.21 % by weight, after which the obtained tissue mass is separated from the solution, treated with distilled water until a pH of 3.0 is obtained and, after separation from the water, mixed again with the distilled water and dispersed up to a particle size not exceeding 0.4 mm.

EP 0 431 164 A1

## PREPARATION OF ANTI-INFLAMMATORY, LACTOGENIC AND STIMULATING EFFECT AND A METHOD OF PRODUCING SAME

Field of the Art

The present invention relates to the veterinary medicine and particularly it applies to a preparation of anti-inflammatory, lactogenic and stimulating effect and a method of its production.

Prior Art

At large pig-breeding complexes sows are indoors and the most part of time they spend in fixed boxes, are deprived of natural physical factors and breeded with monofodder which have been subjected to keeping. As a result of this many animals have difficult delivery, there are often happened post-natal complications that are accompanied by the development of the syndrome of metritis-mastitis-agalactia, by toxicosis and the death of pigs in a farrow.

Literature sources describe different preparations for prophylaxis and treatment of gynaecological diseases in sows, for example, ichthyol-glycerine tampons to be pluged into a vagina, or 20-40 ml 5-10 aqueous ichthyol solutions for intramuscular injections (Veterinary Midwifery and Gynaecology, 1986, Publishing House "Agropromizdat", (Moscow), p. 273), different antibiotics (Veterinary Midwifery and Gynaecology, 1977. Publishing House "Kolos", (Leningrad), p. 336), sulfanilamide and nitrofuran preparations, oxitocine, mammophysium), neutropic preparations (proserinum, carbacholinum), as well as solutions of glucose, calcium gluconate, cordiaminum, coffeine and other preparations, widely used in generally toning up and symptomatic theraulics.

Known in the art preparations do not always yield desirable results. Besides this, the use of antibiotics, as it is wall known, often results in appearing resistant bacterium strains that considerably complicates the duration of the pathological process.

Known in the art are also tissue preparations, produced from a placenta and used in the veterinary gynaecology. The methods of producing these preparations are different, the activity of a preparation depends on a method of its production. For example, according to N.I.Krauze method, the placenta is washed with water from blood and contamination, then it is sectioned into small pieces and concervated in a 2% chloracide solution for 4-5 days. According to the author's data, a placenta can be kept for 6 months. With a medical purpose, the pieces of a concervated placenta are implanted under skin of cows (I.A.Kalashnik, Stimulating Therapeutics in the Veterinary Medicine, 1979, Kiev, pp. 50-51, 76-77). With this purpose placenta suspension is used for injections in the medical practice (M.D.Mashkovsky, Medical Remedies, 1987, Moscow, Publishing House "Medicina", pp. 154-155). The human placenta, concervated under cold conditions, is reduced to fragments and diluted with an isotonic sodium chloride solution. The preparation is sterilized at $120°$ C for an hour. It was injected under skin once 7-10 days; a course needs 3-4 injections.

However, the method of preparation use in the veterinary practice is not handy, as every case of injection needs a surgical operation. In the medical practice the method of preparation use is more handy. However, the preparation is not active enough, as during the process of its production it is put to a long-term thermal treatment.

Summary of the Invention

The patented preparation is new and hasn't been described in the literature.

The object of the present invention is to provide a new intoxic preparation of an anti-inflammatory, lactogenic and stimulating effect for the prophylaxis and the treatment of post-natal gynaecological diseases in sows. Possessing a high effect, allowing to increase piglet safety, as well as to work out a method of its production.

The problem is solved by that, according to the present invention, the patented preparation of an anti-inflammatory, lactogenic and stimulating effect, is an emulsion of the chorion of human placenta denaturated with chlorine in distilled water, pH of which is 2.5-3.0, the size of hard particles is not larger than 0.4 mm, dry residue content is 1.70-1.85% by mass. The emulsion contains nucleic acids not less than 0.05 mg, proteins, not less than 6.0 mg, and hexuronic acids, not less than 0.30 mg in 1 ml of the preparation.

The patented preparation possesses high-effective anti-inflammatory, lactogenic and stimulating action and finds application for the prophylaxis and the treatment of gynaecological diseases in sows.

The use of the patent preparation provides the clinical recovery of 93% sows, treated with it, and the safety of piglets reaches 95%.

The present method of producing the patented preparation is also an object of the invention. In the method of producing the preparation according to the present invention on the basis of human placenta including washing out, reducing and denaturating of the initial stuff, chorion of human placenta is used as the initial stuff, denaturating is fulfilled by the way of treating the initial stuff with a chlorine solution in distilled water, when chlorine concentration in the solution is not higher than 0.21% by mass, then the obtained tissue mass is extracted from the solution, treated with distilled water up to pH 3.0, thereafter water is removed , while the tissue mass is mixed with distilled water and the obtained mix is dispergated up to the particle size not more than 0.4 mm. Denaturating of the initial stuff is preferably carried out at its volume ratio to a chlorine solution, 1:4-6, respectively, during 5 days. Before despergating, the tissue mass is necessary to mix with distilled water at a volume ratio 1:1-1.2, respectively.

Best Mode to Carry out the Invention

The preparation according to the present invention is a yelowish emulsion with particle size not more than 0.4 mm, pH of the emulsion - 2.5-3.0, the dry residue - 1.70-1.85% by mass, proteins, not less than 6.0 mg, nucleic acids, not more than 0.05 mg, and hexuronic acids, mot more than 0.30 mg. The preparation has a specific chlorine smell, is mixed well with water, but with heat or freezing it decomposes. The preparation does not possess incompatibility and volality and it is used as hypodermic and intramuscular injections.

The patented preparation has been experimentally studied on laboratory animals and on sows under production conditions.

The effect of the patented preparation on the reparative processes has been studied in rats with experimental linear wounds and with full layer defects of skin.

The effect of the patented preparation on the healing of linear wounds was studied on 95 male rats, the mass thereof being 240-280 g. There was also determined an effect of the patented preparation on the the strength of rupture of linear wounds (scar strength) using a method of tensiometry. On the cut skin of the back of a rat, narcotized with nambutal (30 mg/kg), there were inflicted 6 sm long linear wounds of all the skin layers under aseptic conditions. Then there were put in three stitches on a wound. The preparations were injected simultaneously when operating and on the fourth day after the operation. On the seventh day the stitches were taken off. On the eighth day the animals were killed by decapitation. With the help of a tensiometer there was determined the strength of an excised skin piece in $g/cm^2$ according to the weight, necessary for the rupture of a new formed scar. Serving as a control there was used placenta suspension.

The test results showed that both in the control animals and in the tested ones there was appeared a small swelling of wound edges just after linear wounds had been inflicted and the stitches had been put in. During the next two days there was observed the healing of the wound by first intention (there was observed neither fester nor inflammation). By the 8-th day of the experiment the scar rose above the skin along the wound in some animals of the control group. It was dense to the touch and partially united with hypodermic tissue, it differed in colour from the surround skin. In the rest animals of the control group these phenomena were expressed to an inconsiderable degree. The weight, needed for the rupture of 1 $cm^2$ skin with a scar in the control animals, was 518.0±57.0 g.

The injection of 2.5 mg patented preparation to the tested animals improved the state of the scar differed from the one of the control animals, as in all the animals it was soft, sleek, elastic and was not united with the hypodermic tissue. It differed a little in colour from the surrounding skin. The scar strength increased 26.4±9.4% (P < 0.1) in these animals. The injection of 31.25 mg placenta suspension (12.5 times as large than the dose of the patented preparation) increased 29.5±8% the scar strength. According to the appearance, the scar in these animals did not differ from the one of the animals injected with the patented preparation. Thus, the patented preparation is more active than placenta suspension in the tests with the experimental linear wounds.

The effect of the patented preparation on the healing of full layer defects of skin was studied.

The experiments were carried out on 72 not pedigree male rats, mass thereof being 240-280 g. It was studied an effect of the patented preparation, in comparison, with placenta suspension, on the healing of full-layer defects of skin, depending on a dose of injected preparation, the time of injection and a defect size. The experiment was fulfilled and treated according to the method of 3x3 "Latin square". The patented preparation was injected to 10 groups of animals, the doses being 5 mg, 12.5 mg, 31.25 mg per an animal. Placenta suspension was injected to 9 groups of animals, the doses being 12.5 mg, 31.25 mg and 78.0 mg per an animal. The preparations were simultaneously injected a week before and a week after a wound was inflicted. The skin defects were 0.5 cm², 1.0 cm², 2.25 cm² and 4.0 cm². The control animals were divided into 4 groups depending on the size of the skin defect, respectively. A full-layer skin piece was cut all over a back skin in narcotized animals (nembutal - 30 mg/kg) according to a stencilled method. The concentration was observed and the full healing of a wound was registrated.

The test results showed that both in the control animals and in the experimental ones a sizable swelling of wound edges was appeared just after a skin piece had been cut all over. During the next days the healing of a wound by first intention took place. To find out suantitative characteristics of the dependence of healing term from an injected dose, the time of injection and a defect size, the mathematical models were constructed.

The injection of both preparations in the tested doses accelerated the healing of full-layer defects of skin, when increasing a dose, the effect became greater. The patented preparation has a considerably higher activity, that results in decreasing the healing term from 20.4±0.8 days (in the control animals that havn't been administered with the preparation) till 16.0±0.09 days, with administration of 0.5 mg preparation per an animal, while placenta suspension induces the reducing of healing term till 16.4±0.03 days when administered in a dose of 78 mg per an animal.

When studying the effect of the time when the preparation was injected, on the healing term, it was shown that the earlier the preparation was injected, the better a wound healing happened. This relationship is more expressed for the patented preparation. Besides this, the introduction of the patented preparetion, inflicted even in later terms, is more effective than any prophylactic injection of placenta suspension.

When the size of a wound is larger in the control animals, the healing term is prolonged. The injection of the patented preparation and placenta suspension accelerates the healing. When injecting the patented preparation, the healing terms in rats, the wounds thereof being 2 cm², decreases 15%, while when injecting placenta suspension, the same effect is observed when the wounds are 0.5 cm². Hence, in these experiments the patented preparation turned out to be considerably more effective than placenta suspension, expecially for extensive skin defects.

Thus, the investigations, carried out on the models of the experimental pathology in animals, have shown that the patented preparation possesses an anti-inflammatory effect to a larger extent than placenta suspension does. It has also a wholesome effect on reparative processes that accelerates wound healing and improve the scar state.

The influence of the patented preparation on sows in farrow and on new-born piglets was studied. 42 sows in farrow were divided into three equal group according to an analogue method two weeks before a farrow. Twice 12-12 days before the farrow and on the day of the farrow all the animals were intramuscularly injected with the patented preparation in different doses, namely the first group was injected with 5 ml, the second one with 7.5 ml and the third one was injected with 10 ml. The general state of the animals was taken into account, the body's temperature was taken; the frequency of respiration and systoles per minute was counted. Besides this, the farrow duration in days was registrates; the duration of foetus removal and the separation of a foetal perimetrium were times; the mass of the perimetrium was determined and there was taken stock of a number of born piglets, including living and stillborn ones, as well as their safety till removal.

The test results showed that at the place the patented preparation had been injected into, there was observed a small, hot, painful swelling. The most marked swelling was in sows of the third group, to which the preparation was injected in a dose of 10 ml. On the third day after the injection of the patented preparation, the swelling disappeared in the animals of the first group, in the rest of the sows the swelling disappeared on the fourth or fifth day after the injection. There were no cases of abscess formed at the places of injection. The general state of the animals was satisfactory. In sows of the second and third groups the rectal temperature increased 0.2-0.4°C and respiration rate increased 3-5 movements per minute 6-12 hours later after the infection of the patented preparation has been inflicted. On the second day these indices became normal.

The patented preparation did not influence the duration of pregnancy. The duration of farrow in sows of all the groups varied within the physiological norm. The test results are given in Table 1 hereinbelow.

Table 1

Effect of the patented preparation on farrow
duration and on the period of foetus removal

| Gro-ups | Dose of prepa-ration, ml | Farrow duration, days | | Foetus removal, min | |
|---|---|---|---|---|---|
| | | M+m | Lim | M+m | Lim |
| 1 | 5.0 | 112.9+0.15 | 112–114 | 127.2+16.2 | 64–200 |
| 2 | 7.5 | 111.5+0.26 | 109–112 | 125.5+15.8 | 65–210 |
| 3 | 10.0 | 112.2+4.08 | 111–117 | 136.0+17.8 | 65–280 |

The duration of foetus removal was registrated from the moment the first piglet was born till the moment the last one was born.

As Table 1 shows, the patented preparation in different doses did not practically have a considerable effect on the duration of foetus removal. These indices were within the same limits as in the rest of the sows at the firm, to which the patented preparation had not been injected.

The test results of the influence of the patented preparation on the time of the removal of foetal membranes and their masses are given in Table 2 hereinbelow. The time of the removal of foetal membranes was marked from the moment the last piglet was born. The foetal membranes were purified from impurities and were weighed.

As Table 2 shows, when injecting, 7.5 ml and 10 ml doses of the patented preparation, the foetal membranes were on the average removed in 100 min, while the use of 5 ml dose of the patented preparation made it possible to remove placenta in 126 min on the average.

Hence , 5 ml dose is not enough, but 10 ml dose does not result in accelerating placenta removal in comparison with 7.5 ml dose.

Table 2

| Gro-ups | Dose of prepa-rati-on, ml | Time of removal of foetal membra-mes, min | | Mass of foetal membranes, g | |
|---|---|---|---|---|---|
| | | M+m | Lim | M+m | Lim |
| 1 | 5.0 | 126.3+30.2 | 40–175 | 2217+163.5 | 1500–3000 |
| 2 | 7.5 | 101.4+20.7 | 15–300 | 2000+164.3 | 1100–2800 |
| 3 | 10.0 | 106.6+21.3 | 40–185 | 2312+285.1 | 1150–3550 |

Thus, 7.5 ml dose is an optimal one of the patented preparation for the influence on the duration of the removal of foetal membranes.

As Table 2 shows, the mass of foetal membranes was the smallest in the II-d group. Evidently, 7.5 ml dose of the patented preparation makes it possible to reduce the mass of foetal membranes owing to the decrease in placenta oedema as well as to the reduction of oedematic phenomena in it.

As Table 3 shows there is a tendency to the increase of a number of living piglets in farrow depending on a dose of the patented preparation. When using 5 ml dose of the patented preparation there was on the average born 10.33 living piglets from one sow, while when using 10 ml 11.2 piglets were born.

Table 3

Results of the effect of the patented preparation
on vitality of nex-born piglets

| No. | Dose of prepara- tion, ml | Piglets born | | | |
|---|---|---|---|---|---|
| | | total | living | stillborn | % of still- born |
| 1 | 2 | 3 | 4 | 5 | 6 |
| 1 | 5.0 | 135 | 124 | 11 | 8.1 |
| 2 | 7.5 | 219 | 189 | 30 | 14.6 |
| 3 | 10.0 | 157 | 146 | 11 | 7.0 |

Table 3 (Cont.)

| No. | Piglets born on the average from one sow | | |
|---|---|---|---|
| | total | living | stillborn |
| 1 | 7 | 8 | 9 |
| 1 | 11.2±0.39 | 10.33±0.59 | 0.92±0.29 |
| 2 | 12.2±0.76 | 10.50±0.50 | 1.73±0.77 |
| 3 | 12.1±0.50 | 11.20±0.48 | 0.85±0.30 |

It is of an important to say that the patented preparation does not increase a number of stillborn piglets. The fact that when using 7.5 ml dose of the patented preparation, the percentage of stillborn piglets was 14.6%,while when using 5 ml and 10 ml it was correspondingly 8.1% and 7%, points out that the patented preparation does not cause the increase in stillborn piglets in the II group. 10 ml dose results even in the lower percentage of stillborn piglets than 5 ml dose does.

There was investigated an effect of the patented preparation on haematopoiesis. Two weeks before farrow 17 sows in farrow were divided according to the analogue method into three groups, i.e. the I group was a control one, it consisted of 5 sows. On the 13-15-th day before farrow and on the day of the farrow there was intramuscularly injected 10 ml isotonic solution of chloride sodium. The II and III groups were experimental ones. Each had 6 sows. At the same terms the experimental groups were intramuscularly injected with 7.5 ml and 10 ml of the patented preparation, correspondingly. All the animals in three groups were under identical conditions of feeding and keeping.

On the day of farrow (13-15 day after the first administration of the patented preparation) as well as on the 6-th and the 16-th day after it, there was taken blood from the sows of the control group and of the tested ones. In blood there was counted a number of erythrocytes and leucocytes, hemoglobin concentration, hematocrite index, erythrocyte sedimentation rate (ESR), a leucocytic picture, the phagocytic activity of leucocytes. Besides this, in a coloured blood smear there was studied a morphological structure of erythrocytes. There was counted an average volume of an erythrocyte and its saturation with hemoglobin as well as the colour index of blood and the index of dislocation of neutrophil granulas.

In serum there was determined the concentration of ascorbic acid (with the help of 2.6-dichlorophenolindophenol)and the activity of aspartic (AST) and alanine (ALT) transaminases. The content of ascorbic acid was also determined in milk and beestings.

There was also made a biochemical urinoscopy (qualitative reactions on sugar, protein, indican, bilirubinum, bilious acids). The test results were worked up according to the method of variational statistics.

The test results showed that in the animals of all the groups, the number of erythrocytes in blood was below the physiologically permissible limits. However, the amount of erythrocytes in blood was greater in the tested animals than in the control ones. This increase was more expressed in the animals of the II group. It should be noted that as far as the investigation term increased after injecting the patented preparation, the number of erythrocytes reduced proportionally in the animals of all the groups. Thus, for example, if in the animals of the I group the first investigation discovered 4.45 mil/mm$^3$ erythrocytes, the second one showed 4.32 mil/mm$^3$ and the third one 4.08 mil/mm$^3$, correspondingly. In the animals of the second group there were 5.80, 5.80, 4.91 mil/mm$^3$ and in the animals of the third group there were observed 4.58, 4.50, 4.15 mil/mm$^3$.

The hematocrite index was lower than normal in the sows of three groups. However, as far as the terms of the investigation increased after injecting the patented preparation, there was observed a tendencyto increasing the hematocrite index in all the groups.

The average value of erythrocytes was larger in the control animals than in the tested ones. When increasing investigation terms after the use of the patented preparation, there was observed an average erythrocytic rise in, the animals of all the groups.

The hemoglobin content in blood of all the animals was within the physiological norm and varied from 5 to 11 g/% during all the periods of the investigation. When fulfilling the first investigation there were found nucleic erythrocytes in all the animals. However, there was only 0.2% in the control sows or it was 33-45 times less than in the tested animals. As to the next two investigations there were not found nucleic erythrocytes in the control animals but in the tested sows there was observed a little amount. Thus; for example, during the first investigation their amount was from 6.6% to 9%, during the second one it was from 1 to 1.2%, and during the third investigation it was from 0.3% to 0.5%. The number of nucleic erythrocytes in distal blood was higher in the animals of the III group than in the ones of the II group.

The erythrocytes with Jolly's bodies were not found in the animals of the control group, while in the tested ones their number was 0.1% when the first investigation was carried out.

The erythrocytes with basophil graininess were not observed in blood of the control sows, their inconsiderable amount was found only in the tested animals when carrying out the first investigation, the animals of the II group had 1.3%, while the sows of the III one had 1.8%.

Large erythrocytes (immature) were found in blood of the animals of three groups, when carrying out all the investigations, however, their number was greater in the tested sows. Thus, for example, if in the animals of the I group the number of large erythrocytes was from 1.5.6% to 18% in distal blood, their amount in the tested sows was greater (from 33.6% to 35%). When increasing the terms of the investigation after using the patented preparation there was observed a proportional reduction of the number of large erythrocytes in distal blood. If during the first investigation the blood contained from 31% to 35% large erythrocytes, during the second one there was from 27.1% to 29.1%, while during the 3-rd investigation the amount of large erythrocytes was greater in blood of the tested sows than in the blood of the control ones.

The content of small (mature) erythrocytes was from 82% to 84% in blood of the control animals. In the tested sows their number was less during all the periods of the investigation, however, when increasing the terms of the investigation after using the patented preparation, there was observed an increase of the number of mature erythrocytes in blood. Thus, for example, when carrying out the first investigation, their amount was from 53% to 60%, during the second investigation 70-71%, and the third - 74-76%. During the third investigation (i.e. a month later the beginning of the use of the patented preparation) the blood of the tested animals had less mature erythrocytes than the control ones.

The patented preparation had also an effect on leucocytes. The number of leucocytes was within the norm in the animals of the I group only when carrying out the first investigation. During the next two investigation as well as in the animals of the II group, the number of leucocytes was insignificantly higher than physiologically permissible limits during all the periods of the investigation. In the sows of the III group, treated with 10 ml patented preparation, the amount of leucocytes was within the limits of the physiological norm during all the periods of the investigation.

The analysis of a leucocytic picture points to the fact that the number of basophil cells was within the norm in the animals of the control group only when carrying out the second investigation. The rest of the cases have showed an increase in basophil cells in all the animals. This increase reached 3-4%.

The eosinophil concentration was within the physiologically permissible limits in the blood of all the animals when carrying out all the investigations.

There were found no myelocytes in blood of the animals during the experiment. The number of immature neutrophiles was increased in the animals during the experiment. When increasing the terms of the investigation after using the patented preparation, there was observed a proportional increase in the content of immature neutrophiles. The number of immature neutrophiles was found to be more expressed in the animals of the control group. Thus, if there was turned out from 2.3% to 7.2% immature neutrophiles in the blood of the tested animals (the norm being up to 2%); in the control sows their content was from 7.4% to 18%, that corresponded to the increase in their content approximately 4-9 times as large as the norm.

The changes in baculinucleic neutrophiles were inconsiderable. During the experiment all the animals of the experimental groups had their content within the limits of the physiological norm, except the sows of the I group during the second investigation, and the ones of the III group during the third investigation, when their increase to 7% was observed.

The content of segment nucleic neutrophiles was within the physiological limits only in the animals of the II and III groups during the first investigation. In all other cases there was found a reduction of their content. In the control animals the reduction was expressed to a greater extent. When increasing the period after the use of the patented preparation the decrease was found to be greater and even during the third investigation the content of segment nucleic neutrophiles in the blood of the tested animals was 2-3 times less than the norm and in the control sows it was 7-7.5 times less.

The lymphocyte quantity was within the physiological limits only in the animals of the tested groups during the first investigation. The lymphocytosis was observed in the rest of the animals. The lymphocytosis was more expressed in the sows of the control group.

The monocyte concentration in blood was within the physiological limits, except the animals of the I group that had a little decrease in the monocyte amount during the second investigation.

The analysis of indicators of the displacement index shoes that only when carrying out the first investigation, this analysis was within the physiological norm in the animals. In the rest of the cases this amount was higher than the norm. Thus, in the sows 3-4 years old the normal index of displacementwas 0.17. The experiments showed the variations in the animals of all groups (the displacement index varried from 0.3 to 2.1), this being an evidence of a considerable rejuvenation of white blood.

The indices of the phagocytic activity of leucocytes is an evidence of the fact that the process of attraction of microbic bodies as regards to neutrophiles develops in different ways. Thus, when carrying out the first investigation, in the control animals the attraction was low, namely 0.8% neutrophiles, surrounded with microbic bodies, while in the tested ones the attraction was high, i.e. in the II group it was 19.5% and in the third one it formed 32.5%. When carrying out the second investigation the attraction increased 8-fold in the animals of the I group and decreased 4.5-fold in the animals of the II group and 7.2-fold in the sows of the III group/ During the third investigation the attraction was high enough and varied from 17% to 21.4%.

The phagocytic activity was high enough in all the animals. At the same time, this index was higher in the animals of the control group than in the ones of the tested groups, except the sows of the III group during the third investigation when the phagocytic activity was higher in the control animals.

During the first investigation the phagocytic index (the average number of microbes phagocyated with one neutrophile) was rather high, namely 16, in the animals of the I group and it was low in the tested ones (in the animals of the II group it was 1.5 and in the sows of the III group it formed 1.8). During the second investigation, the phagocytic index was observed to decrease to 3.2 in the animals of the control group, and this index increased in the tested animals, i.e. it was 8.7 in the II group and 8.5 in the III one. After fulfilling the last blood investigation or 16 days after a farrow, the phagocytic index increased and was 9.6 in the sows of the I group. The phagocytic index in the tested animals of the II group was 4.5 and of the III group 5. The acute and chronic toxicity of the patented preparation was studied. The acute toxicity of the patented preparation was investigated on rabbits and dogs. There were taken 19 rabbits of both sexes, their mass being from 2.5 to 3.5 kg. 10 animals were injected with 30 ml patented preparation (maximum amount permissible for hypodermic injection for rabbits). It was 130 mg/kg of body's mass or it was 100 times as large as the dose, used for sows. The rest of the animals were served as the control ones.

The patented preparation was injected into two dogs, 200 mg for each animals. During the experiment (8 days) in all the animals there was determined their general state (the mobility reflexes, appetite and weight), the rectal temperature and respiration rate. On the 8-th day the tested animals were killed and the macroscopic study of the internal organs was fulfilled. The test results showed that when doing a hypodermic injection of 30 ml patented preparation into the rabbits (about 130 mg/kg) there were observed phenomena of the general excitation. The rectal temperature rose simultaneously. An hour later the injection had been done, the rise of the rectal temperature was observed, 6 hours later it reached its maximum (from $2°$ to $3°$ C as large as the norm) and it became normal 1.5 days later.

At the end of the experiment after the slaughter of an animal there were found no macroscopic changes in their internal organs.

In the acute experiments carried out on two dogs, 200 mg patented preparation were injected into each of the animals (the observation for the animals lasted for 10 days).

The animals showed no changes in the general state, respiration rate and the rectal temperature.

Thus, the study of the acute toxicity in the experiments on the dogs showed that the patented preparation in the doses - (max amount, permissible for a hypodermic injection into this kind of animals) did not cause any toxic reactions, i.e. it did not change their general state, the rectal temperature and respiration rate. In the rabbits the rectal temperature rose after a hypodermic injection. Such a state lasted for 1.5 days; a week later, in all the animals the state was normal and there were nor observed any macroscopic changes in the internal organs.

The study of the chronic toxicity was carried out on 10 rabbits of both sexes. The initial mass of a body was from 2200 g to 3000 g. Before the experiment and till the slaughter, the animals had a normal ration, the room temperature of 16-20° C was held with the help of an air conditioner, the relative humidity was 55-60%. The experiments were carried out during the winter-spring period. Each animal was kept in an individual hutch. There was done a hypodermic injection of the patented preparation in the doses of 2.5 ml (37 mg), i.e. it was 7-15 times larger than the dose used for a sow, and 10 ml (147 mg), i.e. it was greater by 30-60 fold than the fose used for a sow. The test results showed that during 6 months the doses pointed above had no negative effect in the increase of a body's mass. The animals were active, completely ate their feed according to the ration.

The activity of the transaminases (AST and AIT) did not rise in blood serum of the rabbits after 6-month injection of the patented preparation. The main clynic indices i.e. respiration, the rectal temperature and systole rate, according to an electrocardiogram, remained within the limits of the physiological norm. The long-term injection of the patented preparation has no considerable effect on the system of blood coagulation. As regards the morphological blood content , there was observed formation of immature erythrocytes and young neutrophils. The general protein and a proteinogram remained within the limits of the norm.

During the chronic experiment, the functional state of kidneys in the tested was determined with the help of the diurnal diuresis, physico-chemical contents, pH as well as of protein presence in urine. There were no changes in the diurnal diuresis in the tested animals as compared with the control ones. The colour and pellucidness of urine was from light to dark brown. The protein content in urine was the same in the tested animals as in the control ones.

Hence, the long-term injection of the patented preparation had no negative influence on the state of kidneys. The content of the ascorbic acid in adrenal glands and of sugar in blood and urine was used as a test to determine the state of the endocrine system.

The experiments showed that the amount of the ascorbic acid in the adrenal glands of the tested animals did not practically differ from that of the intacted ones.

Hence, the injection of the patented preparation does not effect adversely the function of adrenal glands. The rabbits were killed at the end of the experiment. When dissecting the animals, there were no pathological changes as well as irritating effect on the internal organs. The animal organs were weighed and their weight coefficient was counted (organ weight in ratio to body weight, in %).

The injection of the patented preparation during 6 months does not cause any changes in weight coefficient of the internal organs by comparison with organ coefficient in the control animals.

Thus, the patented preparation hasn't an unhealthy effect on animal organs.

The pathomorphological study of the internal organs in rabbits after the chronic experiment did not show any important changes.

The clinical test was carried out at two sow complexes, 54 thousand pigs each (more than 50 thousand sows) during 5 years.

The patented preparation in 10 ml dose was intramuscularly injected into the middle third of a sow neck. It was injected twice, namely just after a farrow and then 48-72 hours later for a prophylactic purpose. With the treated purpose, the patented preparation was injected 5 times, the interval was the same. When using the patented preparation with a prophylactic purpose, it was found that in the absolute majority of the animals the lochia secretion ended and the changes in the genitals inflammation disappeared on the 5-th-7-th day after a farrow, when the patented preparation had been twice injected. The sows felt healthy, ate well their feed and drank water. The motherly instinct was clearly expressed. At the same time, the injection of the patented preparation into the sows to be sucked positively effected the health and safety of sucking piglets. There was very seldom observed diarrhoea in these farrows. The piglets grew and developed well. In a part of the sows (about 20%) the lochia secretion ended only on the 8th-10th day (after the patented preparation was injected three times). The positive prophilactic effect varied fro 92.4% to 98.8% of the treated animals.

If before using the patented preparation, the main sows farrowed annually with 14 piglets at the pig-breeding complex, after its use this indice rose to 17.8 piglets.

This preparation was used with a clinical purpose on 560 sows that had suppratively-catarrhal secretion from the genitals after a farrow. These animals were registrated to have also symptoms of cardiac failure (obesity, tachycardia, dyspnoea, the peripheral parts of a body became cyanotic) or liver pathology (subicterum, bilirubinurea, the presence of biliary acids in urine). In these farrows the piglets-suckers fell ill with intoxicative gastroenteritis on a mass scale, and many of them died because of this reason. These sows were injected with 10 ml patented preparation 3-5 times, the interval being from 48 to 72 hours. After the injection there was an improvement in the clinical state of the animals. The positive effect was observed in 496 animals (88%).

The patented preparation is produced in the following way.

As an initial raw material the chorion (a part of human placenta) is taken, which is separated from amnion and a navel string, then it is divided into two parts, namely the glabrous and fimbriated ones, the latter is reduced to fragments and then each part is separately kept in water during no less than 1 day (to remove blood remains), changing water every 4-5 hours. Then the tissue mass is denaturated with chlorine solution in distilled water (chlorine concentration is not higher than 0.21% by mass), carefully being mixed an daily pouring out chlorine solution (off the fimbriated chorion through a sieve). The denaturation is carried out in a volume ratio of the initial raw materials to chlorine solution of 1:4-6 correspondingly during 5 days. The whole denaturation is ensured with the help of the solution the chlorine concentration thereof is not higher than 0.21% by mass. The concentration increase results in destruction of the chorion. The denaturated mass is extracted from the solution, mixed with distilled water, to rise pH up to 3.0 and then the mass is isolated from water in a ratio 1:1-1.2 and then each part of dispersed up to the particle size not more than 0.4 mm and intermixed.

The end product produced in an emulsion, with the particle size not more than 0.4 mm. The emulsion contains 1.70-1.85% by mass precipitate and not less than 0.05 mg nucleic acids, 6.0 mg protein and 0.30 mg hexuronic acids in 1 ml.

To understand the invention better, the following examples for producing the patented preparation and of its application are given hereinbelow.

Example 1

The amnion (19 g) and a navel string (33 g) were carefully separated from placenta (450 g), and the remained chorion (398 g) was divided into two parts, namely a fimbriated one (358 g) and a glabrous one (40 g). The fimbriated chorion was grinded and blood was carefully squeezed from it.

Each part was put into filtered water and kept there for a day, every 4 hours the water was changed to achieve the full haemolysis. After that the parts of the fimbriated chorion (340 g) and the glabrous one (35 g) were separately put into a 0.2% chlorine solution in distilled water, the ratio being 1:4, and thoroughly mixed. During 5 days the chlorine solution was drained every day (through a sieve, the diameter of its cells was 1 mm). The fully denaturated mass was separated from the chlorine solution and put into distilled water, the ratio being 1:5, thereby freeing the denaturated mass from chlorine surpluses and rising pH up to 3.0. Then the mass of the glabrous chorion (30 g) and the fimbriated one (325 g) was taken from the water and the two parts were repeatedly treated with distilled water in a ratio 1:1.2 correspondingly. Each part of the mass was grinded till the particle size became 0.4 mm, and then chorion parts were mixed. There was produced 781 g emulsion, containing 1.80% by mass dry residue, 0.05 mg nucleic acids, 6.5 mg protein, 0.35 mg hexuronic acids in 1 ml.

Example 2

The process was carried out according to the one described in Example 1, with the exception that chorion denaturation was carried out with a 0.21% chlorine solution in distilled water, the ratio being 1:6, and before dispersion, the tissue mass was mixed with distilled water in volume ratio 1:1. There was produced 750 g emulsion, containing 1.85% by mass dry residue, 0.055 mg nucleic acids, 7.5 mg protein, 0.35 mg hexuronic acids in 1 ml.

Example 3

The patented preparation was used for treating 14 sows to be sucked with the syndrom of mastitismetritis-agalactia. The sows had 150 piglets-suckers, 146 of which fell ill with hypoglycaemia and colostrum toxicose.

10 ml patented preparation was intramuscularly injected three times, the interval being 2-3 days. The sows and the sucking piglets were under daily clinical observation (namely a body temperature was taken, the number of respiration and pulse rates per a min were calculated, the general clinical state was determined and the general analysis of blood and urea was done). On the 5th-7th day of the treatment the full recovery was observed in all the sows. Simultaneously, already on the 3d day there was observed an improvement of the general state in suckers (they settled down, the defaecation act became rare, the stools had thick consistence, there was no vomiting). The full clinical recovery came on the 4th-5th day of treatment. 139 animals out of 146 ill piglets or 95% were recovered (7 piglets were perisched). These piglets were saved till weaning. There was not observed any relapses in the sows.

5 sows-analogues served as control ones. During the 1st week after a farrow they were registrated to have a syndrom of metritis-mastitis-agalachia accompanied by hypoglycaemia and colostrum toxicose in all the suckers. To treat these sows lactin preparation (the preparation produced from the hypophysis of cattle) was intramuscularly injected in the dose of 200 units twice a day during 5 days. At the same time the animals were intramuscularly injected with 500000 units of streptomycin twice a day during 5 days. The sows had 53 piglets, 50 of them fell ill. The clinical recovery took place in 3 sows on the 7th-9th day, and in one sow on the 12th day. In one sow all the piglets died and this sow was rejected. 16 piglets out of 53 ones were perished (the safety was 70%).

Example 4

The patented preparation was used for prophylactic purposes at a pig-breeding farm. During 5 years there was annually carried out a reckoned prophylactic treatment of all the sows with the patented preparation. The preparation was twice injected, namely 10 ml was injected just after a farrow and then it was repeatedly injected 48-72 hours later. The test results are given in Table 4 hereinbelow.

Table 4

| Indices | Without use of preparation for a year | With the use of the patented preparation | | | | |
|---|---|---|---|---|---|---|
| | | for the 1st year | for the 2-nd year | for the 3-d year | for the 4-th year | for the 5-th year |
| The number of sows to farrow | 5839 | 6379 | 6400 | 6249 | 7351 | 6816 |
| The number of born piglets | 46881 | 53073 | 52790 | 54419 | 56114 | 62843 |
| The number of piglets to be taken from one sow (on the average) | 7.0 | 7.8 | 7.93 | 8.2 | 8.5 | 8.9 |
| The number of all piglets to be taken at the complex) | 40877 | 49959 | 50774 | 51248 | 53037 | 58979 |

Industrial Applicability

The patented preparation of anti-inflammatory, lactogenic and stimulating effect finds application in the veterinary medicine for prophylaxis and treatment of gynaecological deseases in sows.

**Claims**

1. A preparation of anti-inflammatory, lactogenic and stimulating effect characterized by that it is an emulsion of chorion of human placenta, denaturated with chlorine in distilled water, with pH 2.5-3.0, with hard particle size not more than 0.4 mm, with the content of dry residue 1.70-1.85% by mass, containing no less than 0.05 nucleic acids, no less than 6.0 mg protein, no less than 0.30 mg hexuronic acids in 1 ml.

2. A method of producing a preparation of anti-inflammatory, lactogenic and stimulating effect according to Claim 1 on the basis of human placenta, including washing, grinding and denaturation of the initial raw material, characterized by that as an initial material chorion of human placenta is used and the denaturation is carried out by way of treating it with a chlorine solution in distilled water, the chlorine concentration being not higher than 0.2% by mass, then the obtained tissue mass is separated from the solution, and treated with distilled water up to pH 3.0, thereafter removed and the tissue mass is mixed with distilled water and the obtained mixture is dispersed up to the particle size more than 0.4 mm.

3. A method according to Claim 2 characterized by that the denaturation of the initial material is carried out with its volume ratio to a chlorine solution 1:4-6, correspondingly, during 5 days.

4. A method according to any of Claims 2-3 characterized by that before dispersion, the tissue mass is mixed with distilled water at a volume ratio 1:1-1.2, correspondingly.

# INTERNATIONAL SEARCH REPORT

International Application No PCT/SU 89/00106

**I. CLASSIFICATION OF SUBJECT MATTER** (if several classification symbols apply, indicate all) *

According to International Patent Classification (IPC) or to both National Classification and IPC

Int. Cl.$^5$ A61K 35/50

**II. FIELDS SEARCHED**

Minimum Documentation Searched [7]

| Classification System | Classification Symbols |
|---|---|
| Int.Cl.$^4$ | A61K, 17/00, 35/48, 35/50 |

Documentation Searched other than Minimum Documentation
to the Extent that such Documents are Included in the Fields Searched *

**III. DOCUMENTS CONSIDERED TO BE RELEVANT** *

| Category * | Citation of Document, [11] with indication, where appropriate, of the relevant passages [12] | Relevant to Claim No. [13] |
|---|---|---|
| A | GB, B, 1380056 (BEHRINC WERKE AG). 8 January 1975 (08.01.75), the claims | 2-4 |
| | --- | |
| A | US, A, 4054648 (Morinaga Milk Jndustri Co, Ltd) 18 October 1977 (18.10.77) | 2-4 |
| | --- | |
| A | EP, A2, 0302459 (SUMITOMO PHARMACEOTICALS COMPANY LIMITED), 2 February 1989 (02.02.89) & AU, 2028588 JP, 1131199 | 1-4 |

* Special categories of cited documents: [10]

"A" document defining the general state of the art which is not considered to be of particular relevance

"E" earlier document but published on or after the international filing date

"L" document which may throw doubts on priority claim(s) or which is cited to establish the publication date of another citation or other special reason (as specified)

"O" document referring to an oral disclosure, use, exhibition or other means

"P" document published prior to the international filing date but later than the priority date claimed

"T" later document published after the international filing date or priority date and not in conflict with the application but cited to understand the principle or theory underlying the invention

"X" document of particular relevance; the claimed invention cannot be considered novel or cannot be considered to involve an inventive step

"Y" document of particular relevance; the claimed invention cannot be considered to involve an inventive step when the document is combined with one or more other such documents, such combination being obvious to a person skilled in the art.

"&" document member of the same patent family

**IV. CERTIFICATION**

| Date of the Actual Completion of the International Search | Date of Mailing of this International Search Report |
|---|---|
| 23 November 1989 (23.11.89) | 8 January 1990 (08.01.90) |
| International Searching Authority | Signature of Authorized Officer |
| ISA/SU | |

Form PCT/ISA/210 (second sheet) (January 1985)